# EUROPÄISCHE PATENTSCHRIFT

(11) **EP 1 268 436 B1**
(45) Veröffentlichungstag und Bekanntmachung des Hinweises auf die Patenterteilung: **18.08.2004**
(21) Anmeldenummer: 01982438.2
(22) Anmeldetag: 18.10.2001
(51) Int. Cl.: C07D 231/38, A61K 7/13

(54) **NEUE DIAMINOPYRAZOL-DERIVATE UND DIESE VERBINDUNGEN ENTHALTENDE FÄRBEMITTEL**
NOVEL DIAMINOPYRAZOLE DERIVATIVES AND DYES CONTAINING SAID COMPOUNDS
NOUVEAUX DERIVES DE DIAMINOPYRAZOLE ET COLORANTS CONTENANT CES COMPOSES

(30) Priorität: 13.03.2001 DE 10111862
(43) Veröffentlichungstag der Anmeldung: 02.01.2003
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(86) Internationale Anmeldenummer: PCT/EP2001/012061
(87) Internationale Veröffentlichungsnummer: WO 2002/072556

(56) Entgegenhaltungen:
- EP-A- 0 786 244
- DE-U- 20 110 357
- US-A- 6 099 592

## Beschreibung

Die Erfindung betriff neue Diaminopyrazol-Derivate sowie diese Verbindungen enthaltende Mittel zum Färben von Keratinfasern.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 4,5-Diaminopyrazol-Derivate eingesetzt, während als Kupplersubstanzen beispielsweise Resorcin, 4-Chlorresorcin, 1-Naphthol, 3-Aminophenol und Derivate des m-Phenylendiamins zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann. Zur Abdeckung des zunehmend wichtigen Rotbereichs wurde bisher hauptsächlich 4-Aminophenol als Entwickler breit eingesetzt. Die Verwendung von bestimmten Diaminopyrazol-Derivaten und Triaminopyrazol-Derivaten in Oxidationshaarfärbemitteln ist aus der EP-OS 0 740 931, DE-OS 42 34 886, US-A-6,099,592 und EP-OS 0 786 244 bekannt. Diese Verbindungen erfüllen jedoch die an Farbstoffe für Oxidationsfärbemittel gestellten Anforderungen nicht in jeder Hinsicht. So besitzen die erhaltenen Färbungen teilweise keine befriedigende Farbintensität und/oder es werden keine reinen Rottöne erhalten. Es besteht daher weiterhin ein Bedürfnis nach neuen Entwicklersubstanzen, welche die vorgenannten Anforderung in besonderem Maße erfüllen.

Hierzu wurde nun überraschenderweise gefunden, daß neue Diaminopyrazol-Derivate gemäß der allgemeinen Formel (I) die an Entwicklersubstanzen gestellten Anforderungen in besonders hohem Maße erfüllen. So werden unter Verwendung dieser Entwicklersubstanzen mit den meisten bekannten Kupplersubstanzen Farbnuancen erhalten, die außerordentlich farbstark, lichtecht und waschecht sind.

Gegenstand der vorliegende Erfindung sind daher Diaminopyrazol-Derivate der allgemeinen Formel (I) oder deren physiologisch verträgliche, wasserlösliche Salze mit anorganischen oder organischen Säuren 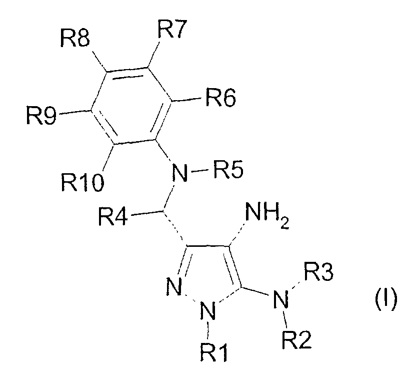 worin
**R1** eine C₁-C₆-Alkylgruppe, eine C₂-C₄-Hydroxyalkylgruppe, eine C₃-C₄-Dihydroxyalkylgruppe oder eine C₂-C₆-Alkoxy-(C₁-C₂)-alkylgruppe, eine Phenylgruppe, eine Benzylgruppe oder eine substituierte Benzylgruppe darstellt;
**R2 und R3** unabhängig voneinander gleich Wasserstoff, einer C₁-C₆-Alkylgruppe, einer Hydroxy(C₂-C₄)alkylgruppe, einer Dihydroxy(C₃-C₄)alkylgruppe oder einer C₂-C₄-Alkoxy-(C₁-C₂)-alkylgruppe sind oder R2 und R3 gemeinsam mit dem Stickstoffatom einen viergliedrigen bis achtgliedrigen heteroaliphatischen Ring bilden;
**R4** Wasserstoff oder eine C₁-C₆ Alkylgruppe darstellt;
**R5** gleich Wasserstoff, einer C₁-C₆-Alkylgruppe, einer ungesättigten C₂-C₆-Alkylgruppe, einer Hydroxy(C₂-C₄)alkylgruppe, einer Dihydroxy(C₃-C₄)alkylgruppe, einer Amino(C₂-C₄)alkylgruppe, einer Dimethylamino(C₂-C₄)alkylgruppe, einer Acetylamino(C₂-C₄)alkylgruppe, einer Methoxy(C₂-C₄)alkylgruppe, einer Ethoxy(C₂-C₄)alkylgruppe, einer C₁-C₄-Cyanoalkylgruppe, einer Carboxy(C₁-C₄)alkylgruppe oder einer Aminocarbonyl(C₁-C₄)alkylgruppe ist;
**R6,R7,R8,R9,R10** unabhängig voneinander Wasserstoff, ein Halogenatom (F,Cl, Br, J), eine Cyanogruppe, eine Hydroxygruppe, eine C₁-C₄-Alkoxygruppe, eine Hydroxy(C₂-C₄)alkoxygruppe, eine C₁-C₆-Alkylgruppe, eine C₁-C₄-Alkylthioethergruppe, eine Mercaptogruppe, eine Nitrogruppe, eine Aminogruppe, eine (C₁-C₄)Alkylaminogruppe, eine Hydroxy(C₂-C₄)alkylaminogruppe, eine Di(C₁-C₄)alkylaminogruppe, eine Di(hydroxy(C₂-C₄)alkyl)aminogruppe, eine (Dihydroxy(C₃-C₄)alkyl)aminogruppe, eine (Hydroxy(C₂-C₄)alkyl)-(C₁-C₄)alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gruppe, eine Hydroxy(C₂-C₄)alkylgruppe oder eine Dihydroxy(C₃-C₄)alkylgruppe bedeuten, oder zwei nebeneinanderliegende Reste R6 bis R10 eine - O-CH2-O-Brücke bilden.

Als Verbindungen der Formel (I) können beispielweise die folgenden Verbindungen genannt werden:
2-Isopropyl-5-phenylaminomethyl-2H-pyrazol-3,4-diamin, 4-[(4,5-Diamino-1-isopropyl-1H-pyrazol-3-ylmethyl)-amino]-phenol, 3-[(4,5-Diamino-1-isopropyl-1H-pyrazol-3-ylmethyl)-amino]-phenol, 5-[(4,5-Diamino-1-isopropyl-1H-pyrazol-3-ylmethyl)-amino]-2-methyl-phenol, 5-[(3,4-Dimethoxy-phenylamino)-methyl]-2-isopropyl-2H-pyrazol-3,4-diamin, 2-[(4,5-Diamino-1-isopropyl-1H-pyrazol-3-ylmethyl)-amino]-4-nitro-phenol, 5-[(3-Amino-phenylamino)-methyl]-2-isopropyl-2H-pyrazol-3,4-diamin, 5-[(4-Amino-phenylamino)-methyl]-2-isopropyl-2H-pyrazol-3,4-diamin, 5-[(4-Amino-2 or 3-methyl-phenylamino)-methyl]-2-isopropyl-2H-pyrazol-3,4-diamin, 2-{5 or 6-Amino-2 or 3-[(4,5-diamino-1-isopropyl-1H-pyrazol-3-ylmethyl)-amino]-phenyl}-ethanol, 2-{4-Amino-2-[(4,5-diamino-1-isopropyl-1 H-pyrazol-3-ylmethyl)-amino]-phenoxy}-ethanol, 5-[(4-Dimethylaminophenylamino)-methyl]-2-isopropyl-2H-pyrazol-3,4-diamin, 2-{4,5-Diamino-3-[(3-amino-phenylamino)-methyl]-pyrazol-1-yl}-ethanol, 2-{4,5-Diamino-3-[(4-amino-phenylamino)-methyl]-pyrazol-1-yl}-ethanol, 2-{4,5-Diamino-3-[(4-amino-2-methyl-phenylamino)-methyl]-pyrazol-1-yl}-ethanol, 2-(4,5-Diamino-3-{[4-amino-2-(2-hydroxy-ethyl)-phenylamino]-methyl}-pyrazol-1-yl)-ethanol, 2-{4,5-Diamino-3-[(4-dimethylamino-phenylamino)-methyl]-pyrazol-1-yl}-ethanol, 2-[4,5-Diamino-3-(benzo[1,3]dioxol-5-ylaminomethyl)-pyrazol-1-yl]-ethanol, 4-Chloro-2-{[4,5-diamino-1-(2-hydroxy-ethyl)-1H-pyrazol-3-ylmethyl]-amino}-phenol, 2-(4,5-Diamino-3-phenylaminomethyl-pyrazol-1-yl)-ethanol, 4-{[4,5-Diamino-1-(2-hydroxyethyl)-1H-pyrazol-3-ylmethyl]-amino}-phenol, 3-{[4,5-Diamino-1-(2-hydroxyethyl)-1H-pyrazol-3-ylmethyl]-amino}-phenol, 5-{[4,5-Diamino-1-(2-hydroxy-ethyl)-1H-pyrazol-3-ylmethyl]-amino}-2-methyl-phenol, 2-{4,5-Diamino-3-[(3,4-dimethoxy-phenylamino)-methyl]-pyrazol-1-yl}ethanol, 2-{[4,5-Diamino-1-(2-hydroxy-ethyl)-1H-pyrazol-3-ylmethyl]-amino}-4-nitro-phenol, 2-(4-Methyl-benzyl)-5-phenylaminomethyl-2Hpyrazol-3,4-diamin, 2-Benzyl-5-phenylaminomethyl-2H-pyrazol-3,4-diamin, 2-Methyl-5-phenylaminomethyl-2H-pyrazol-3,4-diamin, 2-Phenyl-5-phenylaminomethyl-2H-pyrazol-3,4-diamin, 2-t-Butyl-5-phenylaminomethyl-2H-pyrazol-3,4-diamin, 5-[(4-Amino-phenylamino)-methyl]-2-(4-methyl-benzyl)-2H-pyrazol-3,4-diamin, 5-[(4-Amino-phenylamino)-methyl]-2-benzyl-2H-pyrazol-3,4-diamin, 4-[(4,5-Diamino-1-(4-methylbenzyl)-1H-pyrazol-3-ylmethyl)-amino]-phenol, 4-[(4,5-Diamino-1-benzyl-1 H-pyrazol-3-ylmethyl)-amino]-phenol.

Bevorzugt sind Verbindungen der Formel (I), bei denen (i) **R2** und **R3** gleich Wasserstoff sind und **R1** gleich einer C₁-C₄-Alkylgruppe, einer C₂-C₄-Hydroxyalkylgruppe, einer Benzylgruppe oder einer Methylbenzylgruppe ist, und/oder (ii) **R2, R3, R4** und **R5** gleich Wasserstoff sind und **R1** gleich einer C₁-C₄-Alkylgruppe, einer C₂-C₄-Hydroxyalkylgruppe, einer Benzylgruppe oder einer Methylbenzylgruppe ist, und/oder (iii) **R2, R3, R4** und **R5** gleich Wasserstoff sind und **R1** gleich einer C₁-C₄-Alkylgruppe, einer C₂-C₄-Hydroxyalkylgruppe, einer Benzylgruppe oder einer Methylbenzylgruppe ist, und mindestens einer der Reste **R6** bis **R10** gleich einer Hydroxygruppe oder einer Aminogruppe ist während die verbleibenden Reste **R6** bis **R10** gleich Wasserstoff sind.

Insbesondere sind die folgenden Verbindungen oderen Salze mit anorganischen oder organischen Säuren zu nennen:
4-{[4,5-Diamino-1-(2-hydroxy-ethyl)-1H-pyrazol-3-ylmethyl]-amino}-phenol, 2-{4,5-Diamino-3-[(4-amino-phenylamino)-methyl]-pyrazol-1-yl}-ethanol, 2-(4,5-Diamino-3-phenylaminomethyl-pyrazol-1-yl)-ethanol, 2-Isopropyl-5-phenylaminomethyl-2H-pyrazol-3,4-diamin, 2-{4,5-Diamino-3-[(3-aminophenylamino)-methyl]-pyrazol-1-yl}-ethanol, 2-{4,5-Diamino-3-[(4-amino-2-methyl-phenylamino)-methyl]-pyrazol-1-yl}-ethanol, 2-{4,5-Diamino-3-[(4-amino-3-methyl-phenylamino)-methyl]-pyrazol-1-yl}-ethanol, 2-(4,5-Diamino-3-{[4-amino-2-(2-hydroxyethyl)-phenylamino]-methyl}pyrazol-1-yl)-ethanol, 2-(4,5-Diamino-3-{[4-amino-3-(2-hydroxyethyl)-phenylamino]-methyl}-pyrazol-1-yl)-ethanol, 2-{4,5-Diamino-3-[(4-dimethylamino-phenylamino)-methyl]-pyrazol-1-yl}-ethanol, 2-[4,5-Diamino-3-(benzo[1,3]dioxol-5-ylaminomethyl)-pyrazol-1-yl]-ethanol, 4-Chloro-2-{[4,5-diamino-1-(2-hydroxyethyl)-1H-pyrazol-3-ylmethyl]-amino}-phenol, 3-{[4,5-Diamino-1-(2-hydroxyethyl)-1H-pyrazol-3-ylmethyl]-amino}-phenol, 5-{[4,5-Diamino-1-(2-hydroxyethyl)-1H-pyrazol-3-ylmethyl]-amino}-2-methyl-phenol, 2-{4,5-Diamino-3-[(3,4-dimethoxyphenylamino)-methyl]-pyrazol-1-yl}-ethanol, 2-{[4,5-Diamino-1-(2-hydroxyethyl)-1H-pyrazol-3-ylmethyl]-amino}-4-nitro-phenol, 4-[{4,5-Diamino-1-isopropyl-1H-pyrazol-3-ylmethyl)-amino]-phenol, 3-[(4,5-Diamino-1-isopropyl-1H-pyrazol-3-ylmethyl)-amino]-phenol, 5-[(4,5-Diamino-1-isopropyl-1 H-pyrazol-3-ylmethyl)-amino]-2-methyl-phenol, 5-[(3,4-Dimethoxy-phenylamino)-methyl]-2-isopropyl-2H-pyrazol-3,4-diamin, 2-[(4,5-Diamino-1-isopropyl-1H-pyrazol-3-ylmethyl)-amino]-4-nitrophenol, 5-[(3-Amino-phenylamino)-methyl]-2-isopropyl-2H-pyrazol-3,4-diamin, 5-[(4-Amino-phenylamino)-methyl]-2-isopropyl-2H-pyrazol-3,4-diamin, 5-[(4-Amino-2-methyl-phenylamino)-methyl]-2-isopropyl-2Hpyrazol-3,4-diamin, 5-[(4-Amino-3-methyl-phenylamino)-methyl]-2-isopropyl-2H-pyrazol-3,4-diamin, 2-{5-Amino-2-[(4,5-diamino-1-isopropyl-1 H-pyrazol-3-ylmethyl)-amino]-phenyl}-ethanol, 2-{6-Amino-3-[(4,5-diamino-1-isopropyl-1H-pyrazol-3-ylmethyl)-amino]-phenyl}-ethanol, 2-{4-Amino-2-[(4,5-diamino-1-isopropyl-1H-pyrazol-3-ylmethyl)-amino]-phenoxy}-ethanol und 5-[(4-Dimethylamino-phenylamino)-methyl]-2-isopropyl-2H-pyrazol-3,4-diamin.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen Diaminobenzol-Derivate der Formel (I) kann unter Verwendung von bekannten Syntheseverfahren erfolgen. Die Synthese der erfindungsgemäßen Verbindungen kann beispielsweise durch eine reduktive Aminierung eines substituierten Pyrazols der Formel (II) 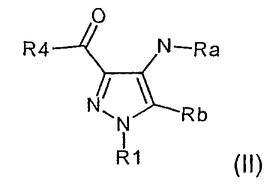 mit einem Amin der Formel (III) 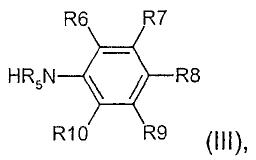 worin
**Ra** die Bedeutung einer Schutzgruppe hat, wie sie zum Beispiel in dem Kapitel "Protective Groups" in Organic Synthesis, Kapitel 7, Wiley Interscience, 1991 beschrieben wird,
**Rb** die Bedeutung NR2Ra oder NR2R3 hat,
und die Reste **R1** bis **R10** die in Formel (I) gennante Bedeutung haben, und anschließende Abspaltung der Schutzgruppe durchgeführt werden.

Die erfindungsgemäßen Diaminopyrazol-Derivate der Formel (I) sind in Wasser gut löslich und ermöglichen Färbungen mit hoher Farbintensität und ausgezeichneter Farbechtheit, insbesondere was die Lichtechtheit, Waschechtheit und Reibeechtheit anbetrifft. Die Verbindungen der Formen (I) weisen weiterhin eine ausgezeichnete Lagerstabilität, insbesondere als Bestandteil der nachfolgend beschriebenen Färbemittel, auf.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein Diaminopyrazol-Derivat der Formel (I) enthalten.

Das Diaminopyrazol-Derivat der Formel (I) ist in dem erfindungsgemäßen Färbemittel in einer Menge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Kupplersubstanzen kommen vorzugsweise N-(3-Dimethylaminophenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxypyridin, 3,5-Diamino-2,6-dimethoxy-pyridin, 1,3-Diamino-benzol, 2,4-Diamino-1-(2-hydroxyethoxy)-benzol, 2,4-Diamino-1-(3-hydroxypropoxy)-benzol, 2,4-Diamino-1-(3-methoxypropoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-2-methyl-phenol, 3-[(2-Hydroxy-ethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-((2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 1,3-Dihydroxy-2,4-dimethyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 4-Hydroxy-indol, 5-Hydroxy-indol, 6-Hydroxy-indol, 7-Hydroxy-indol und 2,3-Indolindion, oder deren Salze, in Betracht.

Obwohl die vorteilhaften Eigenschaften der hier beschriebenen Diaminopyrazol-Derivate der allgemeinen Formel (I) es nahelegen, diese als einzige Entwicklersubstanz zu verwenden, ist es selbstverständlich auch möglich, die Diaminopyrazol-Derivate der allgemeinen Formel (I) gemeinsam mit bekannten Entwicklersubstanzen wie zum Beispiel 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-2,5-dimethyl-benzol, 1,4-Diamino-2,3-dimethyl-benzol, 2-Chlor-1,4-diaminobenzol, 4-Phenylamino-anilin, 4-Dimethylamino-anilin, 4-Diethylaminoanilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1 H-pyrazol, 4,5-Diamino-1-[(4-methyl-phenyl)methyl]-1Hpyrazol, 1-[(4-Chlorphenyl)methyl]-4,5-diamino-1H-pyrazol, 4,5-Diamino-1-methyl-1H-pyrazol, 4,5-Diamino-3-methyl-1-phenyl-1H-pyrazol, 4,5-Diamino-1-(2-hydroxyethyl)-3-methyl-1H-pyrazol, 2-Amino-phenol, 2-Amino-6-methyl-phenol und 2-Amino-5-methyl-phenol oder deren Salzen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß vorhanden sind.

Weiterhin kann das erfindungsgemäße Färbemittel zusätzlich andere Farbkomponenten, beispielsweise 6-Amino-2-methylphenol und 2-Amino-5-methylphenol, enthalten.

Die Verbindungen der Formel (I) können selbstverständlich auch in Kombination mit üblichen direktziehenden anionischen, kationischen, zwitterionischen oder nicht-ionischen Farbstoffen verwendet werden. Zu den bevorzugten anionischen Farbstoffen zählen beispielsweise: 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäure-dinatriumsalz (Cl10316; Acid Yellow No. 1; Food Yellow No. 1), 2-(Indan-1,3-dion-2-yl)chinolin-x,x-sulfonsäure (Gemisch aus Monound Disulfonsäure) (Cl47005;D&C Yellow No. 10; Food Yellow No. 13, Acid Yellow No. 3), 5-Hydroxy-1-(4-sulfophenyl)-4-[(4-sulfophenyl)azo]-pyrazol-3-carbonsäure-trinatriumsalz (Cl19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)amino]-2-phenylamino-benzolsulfonsäure-natriumsalz (Cl10385; Acid Orange No. 3), 4-[(2,4-Dihydroxyphenyl)azo]-benzolsulfonsäuremononatriumsalz (Cl14270; Acid Orange No. 6), 4-[(2-Hydroxynaphth-1-yl)azo]-benzolsulfonsäure-natriumsalz (Cl15510; Acid Orange No. 7), 4-[(2,4-Dihydroxy-3-[(2,4-dimethylphenyl)azo]phenyl)azo]-benzolsulfonsäure-natriumsalz (Cl20170; Acid Orange No. 24), 4-Hydroxy-3-[(4-sulfonaphth-1-yl)azo]-1-naphthalin-sulfonsäure-dinatriumsalz (Cl14720; Acid Red No. 14), 6-Hydroxy-5-[(4-sulfonaphth-1-yl)azo]-2,4-naphthalindisulfonsäure-trinatriumsalz (Cl16255; Ponceau 4R; Acid Red No. 18), 3-Hydroxy-4-[(4-sulfonaphth-1-yl)azol-2,7-naphthalin-disulfonsäuretrinatriumsalz (Cl16185; Acid Red No. 27), 8-Amino-1-hydroxy-2-(phenylazo)-3,6-naphthalin-disulfonsäure-dinatriumsalz (Cl17200; Acid Red No. 33), 5-(Acetylamino)-4-hydroxy-3-[(2-methylphenyl)-azo]-2,7-naphthalin-disulfonsäure-dinatriumsalz (Cl18065; Acid Red No. 35), 2-(3-Hydroxy-2,4,5,7-tetraiod-dibenzopyran-6-on-9-yl)-benzoesäuredinatriumsalz (Cl45430;Acid Red No. 51), N-[6-(Diethylamino)-9-(2,4-disulfophenyl)-3H-xanthen-3-yliden]-N-ethylethanaminium-hydroxid, inneres Salz, Natriumsalz (Cl45100; Acid Red No. 52), 8-[(4-(Phenylazo)-phenyl)azo]-7-naphthol-1,3-disulfonsäure-dinatriumsalz (Cl27290; Acid Red No. 73), 2',4',5',7'-Tetrabrom-3',6'-dihydroxyspiro[isobenzofuran-1(3H),9'-[9H]xanthen]-3-on-dinatriumsalz (Cl45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (Cl45410; Acid Red No. 92), 3',6'-Dihydroxy-4',5'-diiodospiro-[isobenzofuran-1(3H),9'(9H)-xanthen)-3-on-dinatriumsalz (CI45425; Acid Red No. 95), (2-Sulfophenyl)di[4-(ethyl((4-sulfophenyl)methyl)amino)-phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)-phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)phenyl](2,4-disulfophenyl)-carbenium-inneres salz, Natriumsalz (2:1) (CI42045; Food Blue No. 3; Acid Blue No. 1), Bis[4-(diethylamino)phenyl](5-hydroxy-2,4-disulfophenyl)-carbenium-inneres salz, Calciumsalz (2:1) (Cl42051; Acid Blue No. 3), 1-Amino-4-(cyclohexylamino)-9,10-anthrachinon-2-sulfonsäure-natriumsalz (Cl62045; Acid Blue No. 62), 2-(1,3-Dihydro-3-oxo-5-sulfo-2H-indol-2-yliden)-2,3-dihydro-3-oxo-1H-indol-5-sulfonsäuredinatriumsalz (Cl73015; Acid Blue No. 74), 9-(2-Carboxyphenyl)-3-[(2-methylphenyl)-amino]-6-[(2-methyl-4-sulfophenyl)amino]xanthyliuminneres Salz, mononatriumsalz (Cl45190; Acid Violet No. 9), 1-Hydroxy-4-[(4-methyl-2-sulfophenyl)amino]-9,10-anthrachinon-natriumsalz (Cl60730; D&C Violett No. 2; Acid Violet No. 43), Bis[3-nitro-4-[(4-phenylamino)-3-sulfo-phenylamino]-phenyl]-sulfon (Cl10410; Acid Brown No. 13), 5-Amino-4-hydroxy-6-[(4-nitrophenyl)azo]-3-(phenylazo)-2,7-naphthalindisulfonsäure-dinatriumsalz (Cl20470; Acid Black No. 1), 3-Hydroxy-4-[(2-hydroxynaphth-1-yl)azo]-7-nitro-1-naphthalin-sulfonsäure-chromkomplex (3:2) (Cl15711; Acid Black No. 52), 3-[(2,4-Dimethyl-5-sulfophenyl)azo]-4-hydroxy-1-naphthalin-sulfonsäure-dinatriumsalz (Cl14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäuretetranatriumsalz (Cl28440; Food Black No. 1) und 3-Hydroxy-4-(3-methyl-5-oxo-1-phenyl-4,5-dihydro-1H-pyrazol-4-ylazo)-naphthalin-1-sulfonsäure Natriumsalz Chrom-Komplex (Acid Red No. 195).
Zu den bevorzugten kationischen Farbstoffen zählen beispielsweise: 9-(Dimethylamino)-benzo[a]phenoxazin-7-ium-chlorid (CI51175; Basic Blue No. 6), Di[4-(diethylamino)phenyl][4-(ethylamino)naphthyl]carbeniumchlorid (CI42595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)azo]-6-methoxy-3-methylbenzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)-phenyl][4-(methylamino)phenyl]carbenium-chlorid (CI42535; Basic Violet No. 1), Tris(4-amino-3-methylphenyl)-carbenium-chlorid (CI42520; Basic Violet No. 2), Tris[4-(dimethylamino)phenyl]carbenium-chlorid (CI42555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (Cl21010;Basic Brown No. 4), 1-[(4-Aminophenyl)azo]-7-(trimethylammonio)-2-naphtholchlorid (Cl12250; Basic Brown No. 16), 1-[(4-Amino-2-nitrophenyl)azo]-7-(trimethylammonio)-2-naphthol-chlorid (Cl12251; Basic Brown No. 17), 1-[(4-Amino-3-nitro-phenyl)azo]-7-(trimethylammonio)-2-naphthol-chlorid (Cl12251; Basic Brown No. 17), 3,7-Diamino-2,8-dimethyl-5-phenylphenazinium-chlorid (Cl50240; Basic Red No. 2), 1,4-Dimethyl-5-[(4-(dimethylamino)phenyl)azo]-1,2,4-triazolium-chlorid (Cl11055; Basic Red No. 22), 2-Hydroxy-1-[(2-methoxy-phenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)-azo]-pyrazol-5-on-chlorid (CI12719; Basic Yellow No. 57) und Bis[4-(diethyl-amino)phenyl]phenylcarbenium-hydrogensulfat(1:1) (CI42040; Basic Green No. 1).
Als geeignete nichtionische Farbstoffe (insbesondere zum besseren Farbausgleich und zur Erzeugung von speziellen Nuancen) können beispielsweise genannt werden: 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitro-phenol, 1-(2-Hydroxyethoxy)-3-methylamino-4-nitrobenzol, 2,3-(Dihydroxy-propoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino)-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxy-ethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)-amino]-3-nitro-benzonitril (HC Yellow No. 14), 4-[(2-Hydroxyethyl)amino]-3-nitrobenzamid (HC Yellow No. 15), 1-Amino-4-[(2-hydroxyethyl)amino]-2-nitrobenzol (HC Red No. 7), 2-Amino-4,6-dinitro-phenol, 2-Ethylamino-4,6-dinitrophenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxy-ethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitro-benzoesäure, 2-[(4-Amino-2-nitrophenyl)amino]-benzoesäure, 2-Chlor-6-methylamino-4-nitrophenol 2-Chlor-6-[(2-hydroxyethyl)amino]-4-nitro-phenol, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1,4-Di[(2,3-dihydroxypropyl)amino]-9,10-anthrachinon, 1-[(2-Hydroxyethyl)amino]-4-methylamino-9,10-anthrachinon (Cl61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,1 0-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxy-ethyl)amino]-3-methylbenzol (HC Yellow No. 7), 2,6-Diamino-3-[(pyridin-3-yl)azo]-pyridin, 2-((4-(Acetylamino)phenyl)-azo)-4-methylphenol (Cl11855; Disperse Yellow No. 3).

Aus der Gruppe der direktziehenden Farbstoffe sind besonders zu erwähnen: 2-Amino-4,6-dinitrophenol, 2-Ethylamino-4,6-dinitrophenol, 2-[(2-Hydroxy-ethyl)amino]-4,6-dinitrophenol sowie Farbstoffe der allgemeinen Formel (IV), 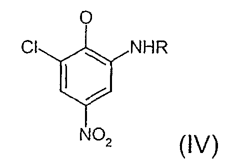 worin R Wasserstoff, Methyl, Ethyl oder Hydroxyethyl bedeutet.

Die Gesamtkonzentration an direktziehenden Farbstoffen beträgt in dem erfindungsgemäßen Mittel etwa 0,1 bis 10 Gewichtsprozent, vorzugsweise etwa 0,1 bis 5 Gewichtsprozent.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Die vorstehend beschriebenen erfindungsgemäßen Kombinationen der Verbindungen der Formel (I) mit oxidativen Haarfarbvorstufen (Entwicklersubstanzen/Kupplersubstanzen) und/oder direktziehenden Farbstoffen werden zur Färbung in einer geeigneten Farbträgermasse appliziert.

Darüber hinaus können in dem Färbemittel noch weitere übliche Zusatzstoffe, beispielsweise Antioxidantien wie Ascorbinsäure, Thioglykolsäure oder Natriumsulfit, sowie Parfümöle, Penetrationsmittel, Puffersysteme, Komplexbildner, Konservierungsstoffe, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,1 bis 30 Gewichtsprozent, die Verdicker in einer Menge von etwa 0,1 bis 30 Gewichtsprozent und die Pflegestoffe in einer Konzentration von etwa 0,1 bis 5,0 Gewichtsprozent.

Das gebrauchsfertige erfindungsgemäße Haarfärbemittel wird durch Mischen der Farbträgermasse mit einem Oxidationsmittel unmittelbar vor der Anwendung hergestellt.

Als Oxidationsmittel kommen hauptsächlich Wasserstoffperoxid oder dessen Additionsverbindungen an Harnstoff, Melamin, Natriumborat oder Natriumcarbonat in Form einer 1- bis 12prozentigen, vorzugsweise einer 3- bis 6prozentigen, wässrigen Lösung, in Betracht. Das Gewichtsverhältnis zwischen Haarfärbemittel und Oxidationsmittel beträgt hierbei vorzugsweise etwa 5:1 bis 1:3, insbesondere 1:1 bis 1:2. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Es ist jedoch prinzipiell auch möglich, zur Oxidation der Farbstoffe anstelle der vorgenannten Oxidationsmittel Luftsauerstoff zu verwenden.

Des pH-Wert des gebrauchsfertigen erfindungsgemäßen Haarfärbemittel stellt sich bei der Mischung der Farbträgermasse (deren pH-Wert etwa gleich 6 bis 11,5 ist) mit dem meist sauer eingestellten Oxidationsmittel (dessen pH-Wert etwa gleich 2 bis 6,5 ist) auf einen pH-Wert ein, der durch die Alkalimengen in der Farbträgermasse und die Säuremengen im Oxidationsmittel sowie durch das Mischungsverhältnis bestimmt wird. Je nach Zusammensetzung kann das erfindungsgemäße Färbemittel schwach sauer, neutral oder alkalisch reagieren und im gebrauchsfertigen Zustand einen pH-Wert von etwa 3 und 11, vorzugsweise etwa 5 bis 10, aufweisen. Die basische Einstellung erfolgt hierbei vorzugsweise mit Ammoniak, wobei jedoch auch organische Amine, zum Beispiel 2-Amino-2-methyl-1-propanol, Tris(hydroxymethyl)amino-methan, Monoethanolamin und Triethanolamin, oder auch anorganische Basen wie Natriumhydroxid und Kaliumhydroxid Verwendung finden. Für eine pH-Wert-Einstellung im sauren Bereich kommen anorganische oder organische Säuren, zum Beispiel Phosphorsäure, Essigsäure, Milchsäure, Ascorbinsäure, Zitronensäure oder Weinsäure, in Betracht.

Anschliessend trägt man eine für die Haarfärbebehandlung ausreichende Menge, je nach Haarfülle, im allgemeinen etwa 60 bis 200 Gramm, dieses Gemisches auf das Haar auf und läßt das Gemisch bei etwa 15 bis 50 Grad Celsius, vorzugsweise 30 bis 40 Grad Celsius, etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Haarfärbemittel mit einem Gehalt an Diaminopyrazol-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt, wobei auch reine Rottöne möglich sind. Die Farbtöne zeichnen sich insbesondere durch ihre hohe Farbintensität aus. Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

### Beispiele

### Beispiel 1: Synthese von 5-Aminomethyl-2H-pyrazol-3,4-diaminen (Allgemeine Synthesevorschrift)

### A. Synthese von Kohlensäure-2-(4,5-Bis-tert-butoxycarbonylaminopyrazol-1-yl)-ethylester-tert-butylester

9,6 g (0,04 mol) 4,5-Diamino-1-(2-hydroxyethyl)pyrazol-sulfat, 11,2 ml Triethylamin und 26,3 g (0,12 mol) Di-tert.-butyl-dicarbonat werden in 500 ml Terahydrofuran gelöst. Die Reaktionmischung wird 58 Stunden lang unter Rückfluss gekocht. Anschließend wird die Reaktionsmischung abgekühlt und durch eine Schicht SiO₂ filtriert. Das Filtrat wird sodann eingeengt und der Rückstand an SiO₂ mit Essigsäureethylester/ Hexan (1:3) chromatographiert.
Es werden 8 g (45 % der Theorie) 2-(4,5-Bis-tert-butoxycarbonylaminopyrazol-1-yl)- carbonsäure-ethylester-tert-butylester erhalten.
¹H-NMR (300 MHz, CDCL3): δ = 7,76 (br,s, 1H); 6,91 (br,s, 1H); 6,51 (br,s, 1 H); 4,35 (m 2H); 4,28 (m, 2H); 1,51-1,47 (m, 27H)

### B. Synthese von [4-tert-Butoxycarbonylamino-2-isopropyl-2H-pyrazol-3-yl]-carbaminsäure-tert.butylester

9,5 g (0,04 mol) 4,5-Diamino-1-isopropyl-pyrazol-sulfat und 17,5 g (0,08 mol) Di-tert.-butyl-dicarbonat werden in einer Mischung von 50 ml 2N Natriunhydrogencarbonat und 120 ml Acetonitril gelöst. Die Reaktionsmischung wird 6 Stunden lang gerührt. Anschließend wird die Reaktionsmischung auf Wasser gegossen und zweimal mit 100ml Essigsäureethylester extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 10 g (73 % der Theorie) [4-tert-Butoxycarbonylamino-2-isopropyl-ethyl)-2H-pyrazol-3-yl]- carbaminsäure-tert.butylester erhalten.
¹H-NMR (300 MHz, CDCL3): δ = 7,48 (s,1H); 6,35 (br,s 1H); 6,10 (br,s 1 H); 4,23 (sp, 1 H); 1,50 (s, 9H); 1,49 (s, 9H); 1,44 (d, 6H)

### C. Synthese von [5-Brom-4-tert-butoxycarbonylamino-2-(2-hydroxyethyl)-2H-pyrazol-3-yl]-carbaminsäure-tert.butylester

Zu einer Lösung von 4,25 g (0,09 mol) 2-(4,5-Bis-tert-butoxycarbonylamino-pyrazol-1-yl)-carbonsäure-ethylester-tert-butylester aus Stufe A wird eine Lösung von 2,13 g N-Brom-succinimid (0,1 mol) in 40 ml Tetrahydrofuran bei Raumtemperatur zugetropft. Das Reaktiongemisch wird 3,5 Stunden lang bei Raumtemperatur gerührt und sodann auf eine Mischung aus 100 ml wässerige Na₂S₂O₃-Lösung und 100 ml Essigsäureethylester gegossen. Die wässerige Phase wird mit Essigsäureethylester extrahiert, die vereinigten organischen Phasen werden sodann mit gesättigter Kochsalzlösung gewaschen, getrocknet und eingeengt. Der Rückstand wird in einer Mischung aus 15 ml Methanol, 50 ml Hexan und 15 ml Wasser aufgenommen und auf 4 °C gekühlt. Anschließend wird das Reaktiongemisch portionsweise mit 10 Äquivalenten Lithiumhydroxid versetzt, über Nacht bei Raumtemperatur gerührt und sodann auf eine 1:1-Mischung einer 5%-igen wässerigen Natriumhydrogenphosphat Lösung mit Essigsäureethylester gegossen. Die organische Phase wird getrocknet, eingeengt und der Rückstand an SiO2 mit einem Gradienten von Essigester/Hexan (1:1 to 2: 1) chromatographiert. Es werden 3,3 g (78 % der Theorie) [4-tert-Butoxycarbonylamino-5-brom-2-isopropyl-2Hpyrazol-3-yl]-carbaminsäure-tert.butylester erhalten.
¹H-NMR (300 MHz, CDCL3): δ = 7,19 (br,s 1H); 6 (br,s 1H); 4,12 (m, 2H), 3,99 (m, 2H); 2,04 (s, 1 H); 1,50 (s, 9H); 1,49 (s, 9H)

### D. Synthese von [4-tert-Butoxycarbonylamino-5-brom-2-isopropyl-2Hpyrazol-3-yl]-carbaminsäure-tert.butylester

Zu einer Lösung von 5,5 g (0,016 mol) [4-tert-Butoxycarbonylamino-2-isopropyl-2H-pyrazol-3-yl]-carbaminsäure-tert.butylester aus Stufe B wird eine Lösung von 3,5 g (0,02 mol) N-Brom-succinimid in 40 ml Tetrahydrofuran bei Raumtemperatur zugetropft. Das Reaktiongemisch wird 3,5 Stunden lang bei Raumtemperatur gerührt und sodann auf eine Mischung aus 100 ml wässerige Na₂S₂O₃-Lösung und 100 ml Essigsäureethylester gegossen. Die wässerige Phase wird mit Essigsäureethylester extrahiert, die vereinigten organischen Phasen mit gesättigter Kochsalzlösung gewaschen, getrocknet und eingeengt. Der Rückstand wird in 100 ml Diethylether/Hexan (1:1) suspendiert, filtriert und getrocknet. Es werden 6 g (89 % der Theorie) [4-tert-Butoxycarbony-lamino-5-brom-2-isopropyl-2Hpyrazol-3-yl]- carbaminsäure-tert.butylester erhalten.
¹H-NMR (300 MHz, CDCL3): δ = 6,77 (br,s 1H); 5,95 (br,s 1H); 4,46 (sp, 1 H), 1,50 (s, 9H); 1,49, s, 9H); 1,44 (d, 6H)

### E. Synthese von [4-tert-Butoxycarbonylamino-5-formyl-2-isopropyl-2Hpyrazol-3-yl]-carbaminsäure-tert.butylester

3,7 g (0,01 mol) [4-tert-Butoxycarbonylamino-5-brom-2-isopropyl-2Hpyrazol-3-yl]-carbaminsäure-tert.butylester aus Stufe D werden unter Argon in 100 ml wasserfreiem Tetrahydrofuran gelöst. Schrittweise werden 17 ml einer 1,6 molaren etherischen Methyllithiumlösung (0,03 mol) zugegeben. Die Reaktionsmischung wird auf -78 °C abgekühlt und schrittweise mit 7 ml einer 1,5molaren tert.-Butyllithiumlösung (0,01 mol) versetzt. Nach beendeter Zugabe wird die Lösung noch 30 Minuten lang bei der angegebenen Temperatur gerührt. Anschließend werden 1,2 g (0,02 mol) Dimethylformamid zugegeben und die Reaktionmischung wird eine Stunde lang bei -78 °C gerührt. Nach langsamer Erwämung auf Raumtemperatur wird die Reaktionsmischung mit Wasser hydrolisiert und dann auf Diethylether gegossen, die wässerige Phase mit Diethylether extrahiert und sodann die vereinigten organischen Phasen mit Magnesiumsulfat getrocknet. Das Lösungsmittel wird am Rotationsverdampfer abdestilliert und der Rückstand an Kieselgel mit Petrolether/Essigsäureethylester (9:1) gereinigt.
¹H-NMR (300 MHz, CDCL3): δ = 9,86 (s,1H); 7,57 (br,s 1H); 7,33 (br,s 1 H); 4,61 (sp, 1 H), 1.50 (s, 9H); 1,49, s, 9H); 1,47 (d, 6H)

### F. Synthese von [4-tert-Butoxycarbonylamino-5-formyl-2-(2-hydroxyethyl)-2H-pyrazol-3-yl]-carbaminsäure-tert.butylester

4,21 g (0,01 mol) [5-Brom-4-tert-Butoxycarbonylamino-2-(2-hydroxyethyl)-2H-pyrazol-3-yl]- carbaminsäure-tert.butylester aus Stufe C werden unter Argon in 100 ml wasserfreiem Tetrahydrofuran gelöst. Schrittweise werden 17 ml einer 1,6molaren etherischen Methyllithiumlösung (0,03 mol) zugegeben. Die Reaktionsmischung wird auf -78°C gekühlt, 7 ml einer 1,5molaren t-Butyllithiumlösung (0,01 mol) werden noch schrittweise zugegeben. Nach beendeter Zugabe wird die Lösung noch 30 Minuten bei der angegebenen Temperatur gerührt. Anschließend werden 1,2 g Dimethylformamid (0.02 mol) zugegeben und die Reaktionmischung wird eine Stunde bei -78°C gerührt. Nach langsamer Erwämung auf Raumtemperatur wird die Reaktion mit Wasser hydrolisiert und dann auf Diethylether gegossen. Anschließend wird die wässerige Phase mit Diethylether extrahiert und sodann werden die vereinigten organischen Phasen mit Magnesiumsulfat getrocknet. Das Lösungsmittel wird am Rotationsverdampfer abdestilliert und der Rückstand an Kieselgel mit Petrolether/Essigsäureethylester (9:1) gereinigt.
¹H-NMR (300 MHz, CDCL3): δ = 9,86 (s, 1H); 7,6 (br,s 2H); 4,29 (m, 2H); 4,10 (m, 2H); 1.50 (s, 9H); 1,50 (s, 9H); 1,48 (d, 6H)

### G. Synthese von 5-aminomethyl-2H-pyrazol-3,4-diaminen

0,0001 mol des Aldehydderivates aus Stufe E oder F und 0,00015 mol des entsprechenden Amins werden in 1,2-Dichlorethan gelöst. Anschließend werden 0,1 ml einer Essigsäurelösung (1 M in 1,2-Dichlorethan) und 0,06 g NaBH(OAc)₃ (0,0003 mol) zugegeben und die Reaktionsmischung wird 5 bis 15 Stunden bei Raumtemperatur gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt. Anschließend werden zur Herstellung des Hydrochlorides 1,5 ml einer 2,9molaren ethanolische Salzsäurelösung zugetropft. Der Niederschlag wird abfiltriert, zweimal mit 1 ml Ethanol gewaschen und sodann getrocknet.

### a. 4-{[4,5-Diamino-1-(2-hydroxyethyl)-1H-pyrazol-3-ylmethyl]-amino}phenol∗HCl

Verwendetes Aldehydderivat: gemäß Stufe **F**
Verwendetes Amin: 4-Amino-phenol
Massenspektrum: MH⁺ 264 (100)

### b. 2-{4,5-Diamino-3-[(4-amino-phenylamino)-methyl]-pyrazol-1-yl}ethanol∗HCl

Verwendetes Aldehydderivat: gemäß Stufe **F**
Verwendetes Amin: 1,4-Diamino-benzol
Massenspektrum: MH⁺ 263 (100)

### c. 2-(4,5-Diamino-3-phenylaminomethyl-pyrazol-1-yl)-ethanol∗HCl

Verwendetes Aldehydderivat: gemäß Stufe **F**
Verwendetes Amin: 1,3-Diamino-benzol
Massenspektrum: MH⁺ 248 (100)

### d. 2-Isopropyl-5-phenylaminomethyl-2H-pyrazol-3,4-diamin∗HCl

Verwendetes Aldehydderivat: gemäß Stufe **F**
Verwendetes Amin: Anilin
Massenspektrum: MH⁺ 246 (100)

### e. 2-{4,5-Diamino-3-[(3-amino-phenylamino)-methyl]-pyrazol-1-yl}ethanol∗HCl

Verwendetes Aldehydderivat: gemäß Stufe **F**
Verwendetes Amin: 1,3-Diamino-benzol
Massenspektrum: MH⁺ 263 (100)

### f. 2-{4,5-Diamino-3-[(4-amino-2-methyl-phenylamino)-methyl]-pyrazol-1-yl}-ethanol∗HCl und 2-{4,5-Diamino-3-[(4-amino-3-methyl-phenylamino)-methyl]-pyrazol-1-yl}-ethanol∗HCl

Verwendetes Aldehydderivat: gemäß Stufe **F**
Verwendetes Amin: 2,5-Diamino-toluol
Massenspektrum: MH⁺ 277 (100)

### g. 2-(4,5-Diamino-3-{[4-amino-2-(2-hydroxyethyl)-phenylamino]-methyl}-pyrazol-1-yl)-ethanol∗HCl und 2-(4,5-Diamino-3-{[4-amino-3-(2-hydroxyethyl)-phenylamino]-methyl}-pyrazol-1-yl)-ethanol*HCl

Verwendetes Aldehydderivat: gemäß Stufe **F**
Verwendetes Amin: 2-(2,5-Diamino-phenyl)-ethanol
Massenspektrum: MH⁺ 307 (100)

### h. 2-{4,5-Diamino-3-[(4-dimethylamino-phenylamino)-methyl]-pyrazol-1-yl}-ethanol∗HCl

Verwendetes Aldehydderivat: gemäß Stufe **F**
Verwendetes Amin: 4-Dimethylamino-anilin
Massenspektrum: MH⁺ 291 (100)

### i. 2-[4,5-Diamino-3-(benzo[1,3]dioxol-5-ylaminomethyl)-pyrazol-1-yl]-ethanol∗HCl

Verwendetes Aldehydderivat: gemäß Stufe **F**
Verwendetes Amin: Benzo[1,3]dioxol-5-ylamine
Massenspektrum: MH⁺ 292 (100)

### j. 4-Chloro-2-{[4,5-diamino-1-(2-hydroxyethyl)-1H-pyrazol-3-ylmethyl]-amino}-phenol∗HCl

Verwendetes Aldehydderivat: gemäß Stufe **F**
Verwendetes Amin: 2-Amino-4-chlor-phenol
Massenspektrum: MH⁺ 297 (100)

### k. 3-{[4,5-Diamino-1-(2-hydroxyethyl)-1H-pyrazol-3-ylmethyl]-amino}phenol∗HCl

Verwendetes Aldehydderivat: gemäß Stufe **F**
Verwendetes Amin: 3-Amino-phenol
Massenspektrum: MH⁺ (100)

### I. 5-{[4,5-Diamino-1-(2-hydroxyethyl)-1H-pyrazol-3-ylmethyl]-amino}-2-methyl-phenol∗HCl

Verwendetes Aldehydderivat: gemäß Stufe **F**
Verwendetes Amin: 5-Amino-2-methyl-phenol
Massenspektrum MH⁺ 264 (100)

### m. 2-{4,5-Diamino-3-[(3,4-dimethoxy-phenylamino)-methyl]-pyrazol-1-yl}-ethanol∗HCl

Verwendetes Aldehydderivat: gemäß Stufe **F**
Verwendetes Amin: 3,4-Dimethoxy-anilin
Massenspektrum: MH⁺ 308 (100)

### n. 2-{[4,5-Diamino-1-(2-hydroxyethyl)-1H-pyrazol-3-ylmethyl]-amino}-4-nitro-phenol∗HCl

Verwendetes Aldehydderivat: gemäß Stufe **F**
Verwendetes Amin: 2-Amino-4-nitro-phenol
Massenspektrum: MH⁺ 309 (100)

### o. 4-[(4,5-Diamino-1-isopropyl-1H-pyrazol-3-ylmethyl)-amino]-phenol∗HCl

Verwendetes Aldehydderivat: gemäß Stufe **E**
Verwendetes Amin: 4-Amino-phenol
Massenspektrum: MH⁺ 262 (100)

### p. 3-[(4,5-Diamino-1-isopropy)-1H-pyrazol-3-ylmethyl)-amino]-phenol∗HCl

Verwendetes Aldehydderivat: gemäß Stufe **E**
Verwendetes Amin: 3-Amino-phenol
Massenspektrum: MH⁺ 262 (100)

### q. 5-[(4,5-Diamino-1-isopropyl-1H-pyrazol-3-ylmethyl)-amino]-2-methyl-phenol∗HCl

Verwendetes Aldehydderivat: gemäß Stufe **E**
Verwendetes Amin: 5-Amino-2-methyl-phenol
Massenspektrum: MH⁺ (100)

### r. 5-[(3,4-Dimethoxy-phenylamino)-methyl]-2-isopropyl-2H-pyrazol-3,4-diamin∗HCl

Verwendetes Aldehydderivat: gemäß Stufe **E**
Verwendetes Amin: 3,4-Dimethoxy-anilin
Massenspektrum: MH⁺ 306 (100)

### s. 2-[(4,5-Diamino-1-isopropyl-1H-pyrazol-3-ylmethyl)-amino]-4-nitrophenol∗HCl

Verwendetes Aldehydderivat: gemäß Stufe **E**
Verwendetes Amin: 2-Amino-4-nitro-phenol
Massenspektrum: MH⁺ 306 (40)

### t. 5-[(3-Amino-phenylamino)-methyl]-2-isopropyl-2H-pyrazol-3,4-diamin∗HCl

Verwendetes Aldehydderivat: gemäß Stufe **E**
Verwendetes Amin: 1,3-Diamino-benzol
Massenspektrum: MH⁺ 261 (100)

### u. 5-[(4-Amino-phenylamino)-methyl]-2-isopropyl-2H-pyrazol-3,4-diamin∗HCl

Verwendetes Aldehydderivat: gemäß Stufe **E**
Verwendetes Amin: 1,4-Diamino-benzol
Massenspektrum: MH⁺ 261 (100)

### v. 5-[(4-Amino-2-methyl-phenylamino)-methyl]-2-isopropyl-2H-pyrazol-3,4-diamin*HCl und 5-[(4-Amino-3-methyl-phenylamino)-methyl]-2-isopropyl-2H-pyrazol-3,4-diamin∗HCl

Verwendetes Aldehydderivat: gemäß Stufe **E**
Verwendetes Amin: 2,5-Diamino-toluol
Massenspektrum: MH⁺ 275 (100)

### w. 2-{5-Amino-2-[(4,5-diamino-1-isopropyl-1H-pyrazol-3-ylmethyl)-amino]-phenyl}-ethanol∗HCl und 2-{6-Amino-3-[(4,5-diamino-1-isopropyl

### 1H-pyrazol-3-ylmethyl)-amino]-phenyl}-ethanol∗HCl

Verwendetes Aldehydderivat: gemäß Stufe **E**
Verwendetes Amin: 2-(2,5-Diamino-phenyl)-ethanol
Massenspektrum: MH⁺ 305 (100)

### x. 2-{4-Amino-2-[(4,5-diamino-1-isopropyl-1H-pyrazol-3-ylmethyl)-amino]-phenoxy}-ethanol∗HCl

Verwendetes Aldehydderivat: gemäß Stufe **E**
Verwendetes Amin: 2-(2,4-Diamino-phenoxy)-ethanol
Massenspektrum: MH⁺ 321 (100)

### y. 5-[(4-Dimethylamino-phenylamino)-methyl]-2-isopropyl-2H-pyrazol-3,4-diamin∗HCl

Verwendetes Aldehydderivat: gemäß Stufe **E**
Verwendetes Amin: 4-Dimethylamino-anilin
Massenspektrum: MH⁺ 289 (100)

### Beispiel 2 bis 26: Haarfärbemittel

Es werden Haarfärbelösungen der folgenden Zusammensetzung hergestellt:

| | |
|---|---|
| 1,25 mmol | Entwicklersubstanz der Formel (I) gemäß Tabelle 1 |
| 1,25 mmol | Kupplersubstanz gemäß Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel Nr.** | **Entwicklersubstanz der Formel (I)** | **Kupplersubstanz** | | | |
|---|---|---|---|---|---|
| | | **I.** **3-Amino- 2-chlor-6-methylphenol** | **II.** **1,3-Diamino-4-(2-hydroxyethoxy)-benzol-sulfat** | **III.** **5-Amino-2-methylphenol** | **IV.** **1-Naphtol** |
| **2.** | Gemäß Beispiel **1a** | rot | violett | orange | hellorange |
| **3.** | gemäß Beispiel **1b** | violett | blau | weinrot | graurot |
| **4.** | gemäß Beispiel **1c** | violett | blauviolet | rot | orange |
| **5.** | gemäß Beispiel **1d** | violett | blauviolet | rot | orange |
| **6.** | gemäß Beispiel **1e** | violett | blau | weinrot | graurot |
| **7.** | gemäß Beispiel **1f** | violett | blau | weinrot | graurot |
| **8.** | gemäß Beispiel **1g** | violett | blau | weinrot | graurot |
| **9.** | gemäß Beispiel **1h** | hell-violett | hellblau | weinrot | graurot |
| **10.** | gemäß Beispiel **1i** | violett | blauviolet | rot | orange |
| **11.** | gemäß Beispiel **1j** | hell-violett | hellblau | weinrot | graurot |
| **12.** | gemäß Beispiel **1k** | hell-violett | hellblau | weinrot | graurot |
| **13.** | gemäß Beispiel **1l** | hell-violett | hellblau | weinrot | graurot |
| **14.** | gemäß Beispiel **1m** | hell-violett | hellblau | rot | graurot |
| **15.** | gemäß Beispiel **1n** | rot | braun | orange | orange |
| **16.** | gemäß Beispiel **1o** | rot | violett | orange | hellorange |
| **17.** | gemäß Beispiel **1p** | violett | violettblau | weinrot | orange |
| **18.** | gemäß Beispiel **1q** | hell-violett | hellblau | weinrot | orange |
| **19.** | gemäß Beispiel **1r** | hell-violett | hellblau | weinrot | orange |
| **20.** | gemäß Beispiel **1s** | hell-violett | hellblau | weinrot | orange |
| **21.** | gemäß Beispiel **1t** | hell-violett | hellblau | weinrot | orange |
| **22.** | gemäß Beispiel **1u** | violett | blau | hellviolett | grau |
| **23.** | gemäß Beispiel **1v** | violett | blau | hellviolett | grau |
| **24.** | gemäß Beispiel **1w** | violett | blau | hellviolett | grau |
| **25.** | gemäß Beispiel **1x** | hell-violett | hellblau | hellviolett | grau |
| **26.** | gemäß Beispiel **1y** | hell-violett | hellblau | hellviolett | grau |

### Beispiel 27 bis 38: Haarfärbemittel

Es werden Haarfärbelösungen der folgenden Zusammensetzung hergestellt:

| | |
|---|---|
| X g | Entwicklersubstanz **E1** bzw. **E2** der Formel (I) gemäß Tabelle 2 |
| U g | Entwicklersubstanz **E8** bis **E15** gemäß Tabelle 2 |
| Y g | Kupplersubstanz **K11** bis **K36** gemäß Tabelle 4 |
| 10,0 g | Kaliumoleat (8prozentige wässrige Lösung) |
| 10,0 g | Ammoniak (22-prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

### Beispiel 39 bis 50: Haarfärbemittel

Es werden cremeförmige Farbträgermassen der folgenden Zusammensetzung hergestellt:

| | |
|---|---|
| X g | Entwicklersubstanz **E3** bis **E6** der Formel (I) gemäß Tabelle 2 |
| U g | Entwicklersubstanz **E8** bis **E15** gemäß Tabelle 2 |
| Y g | Kupplersubstanz **K11** bis **K36** gemäß Tabelle 4 |
| Z g | direktziehender Farbstoff **D2** gemäß Tabelle 3 |
| 15,0 g | Cetylalkohol |
| 0,3 g | Ascorbinsäure |
| 3,5 g | Natriumlaurylalkoholdiglycolethersulfat, 28prozentige wässrige Lösung |
| 3,0 g | Ammoniak, 22prozentige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6prozentigen Wasserstoffperoxidlösung vermischt. Anschließend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 6 zusammengefasst.

**Tabelle 2:**

| **Entwicklersubstanzen** | |
|---|---|
| **E1** | 4-{[4,5-Diamino-1-(2-hydroxy-ethyl)-1H-pyrazol-3-ylmethyl]-amino}-phenol∗HCl |
| **E2** | 4-[(4,5-Diamino-1-isopropyl-1H-pyrazol-3-ylmethyl)-amino]-phenol∗HCl |
| **E3** | 2-{4,5-Diamino-3-[(4-amino-phenylamino)-methyl]-pyrazol-1-yl}-ethanol∗HCl |
| **E4** | 5-[(4-Amino-phenylamino)-methyl]-2-isopropyl-2H-pyrazol-3,4-diamin∗HCl |
| **E5** | 2-Isopropyl-5-phenylaminomethyl-2H-pyrazol-3,4-diamin∗HCl |
| **E6** | 2-(4,5-Diamino-3-phenylaminomethyl-pyrazol-1-yl)-ethanol∗HCl |
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol∗2HCl |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin∗2HCl |
| **K17** | 2,4-Diamino-5-ethoxy-toluol-sulfat |
| **K18** | N-(3-Dimethylamino)phenylharnstoff |
| **K19** | 1,3-Bis(2,4-Diaminophenoxy)propan∗4HCl |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol∗HCl |
| **K35** | 3,4-Methylendioxy-phenol |
| **K36** | 2-Amino-5-methyl-phenol |

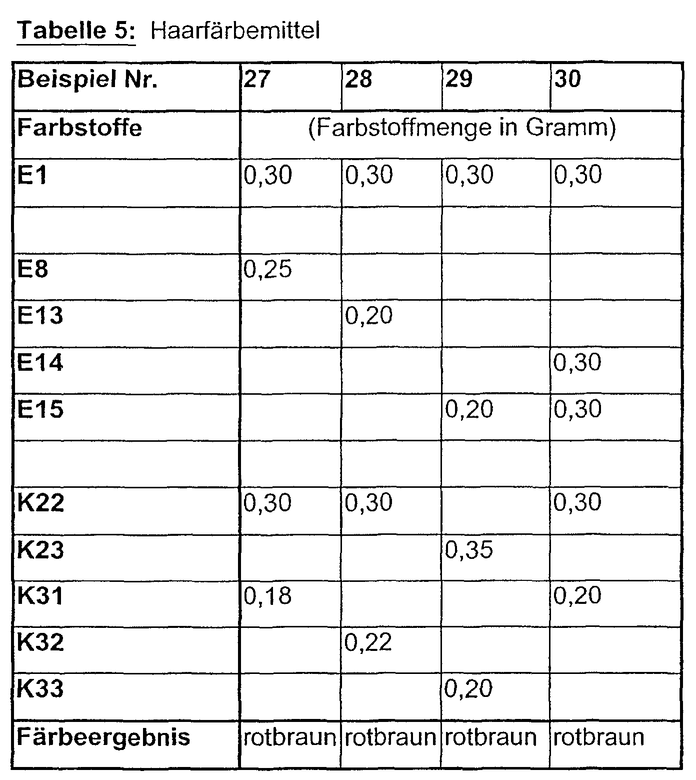

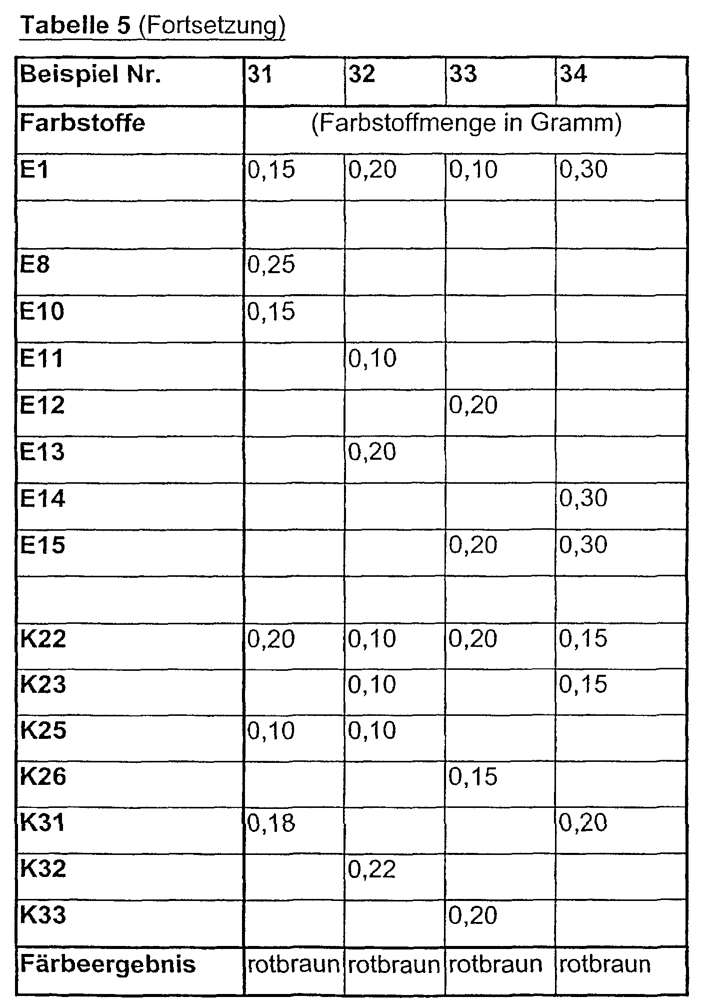

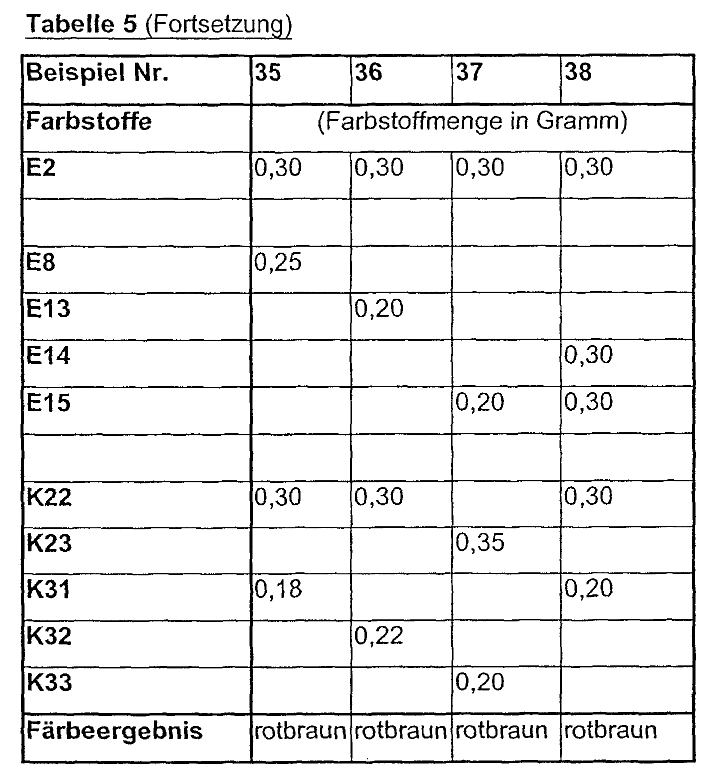

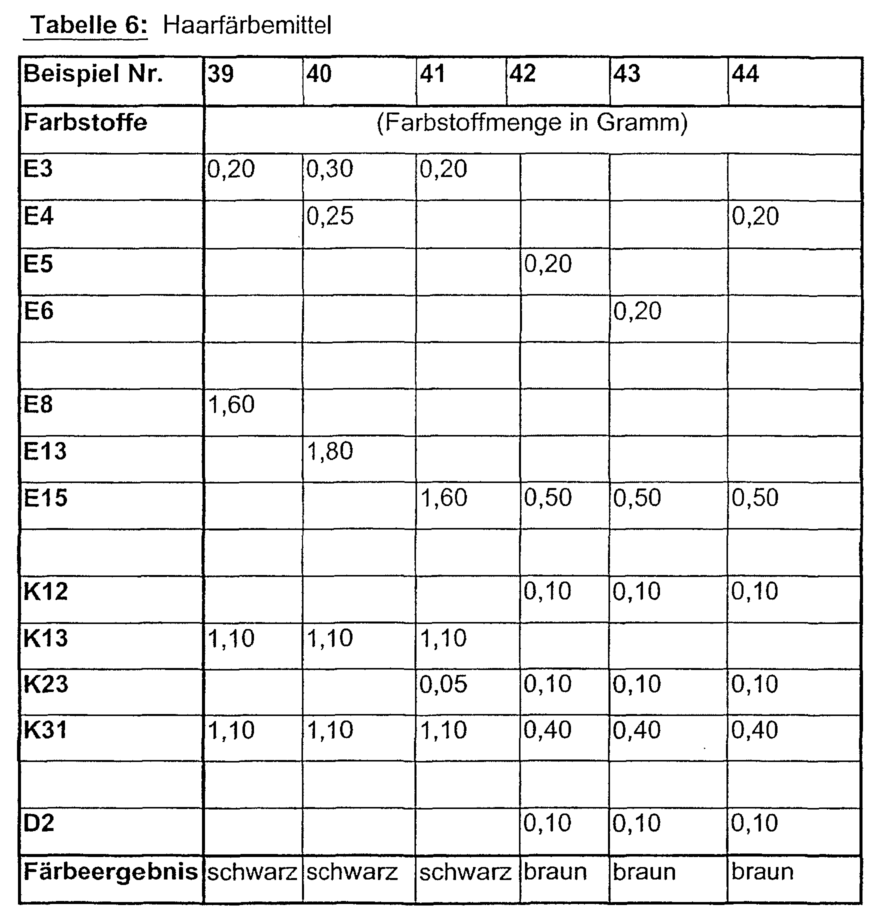

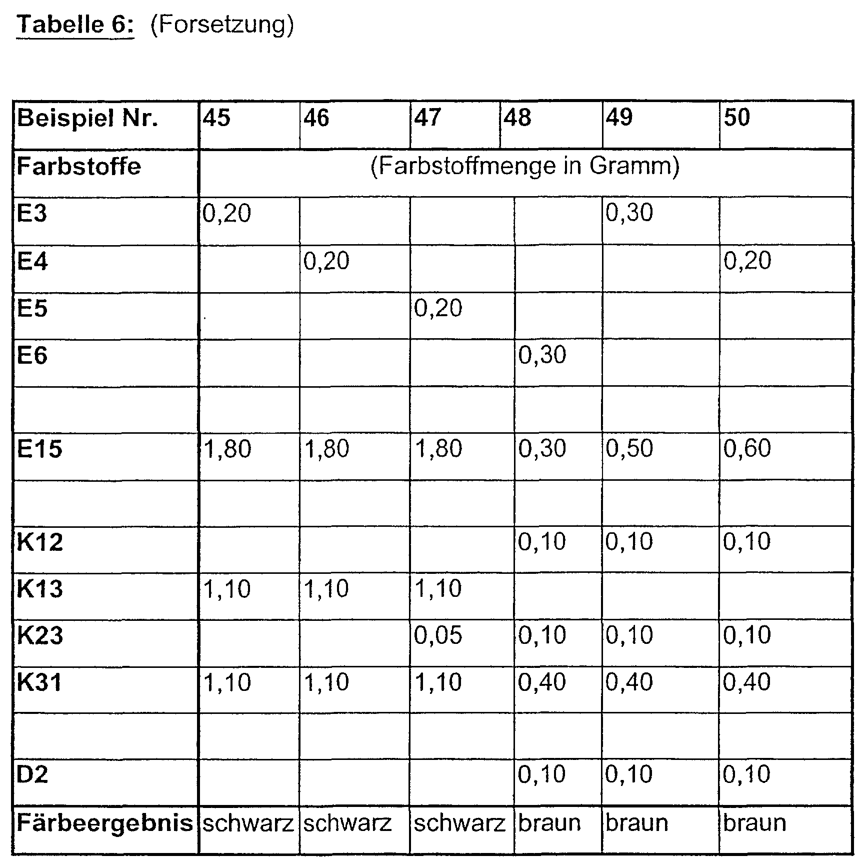

Alle in der vorliegenden Anmeldung enthaltenen Prozentangaben stellen soweit nicht anders angegeben Gewichtsprozente dar.

## Patentansprüche

1. Diaminopyrazol-Derivat der allgemeinen Formel (I) oder dessen physiologisch verträgliche, wasserlösliche Salze mit anorganischen oder organischen Säuren 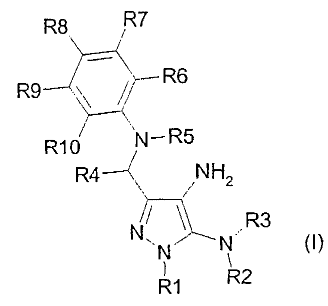 worin
**R1** eine C₁-C₆-Alkylgruppe, eine C₂-C₄-Hydroxyalkylgruppe, eine C₃-C₄-Dihydroxyalkylgruppe oder eine C₂-C₆-Alkoxy-(C₁-C₂)-alkylgruppe, eine Phenylgruppe, eine Benzylgruppe oder eine substituierte Benzylgruppe darstellt;
**R2 und R3** unabhängig voneinander gleich Wasserstoff, einer C₁-C₆-Alkylgruppe, einer Hydroxy(C₂-C₄)alkylgruppe, einer Dihydroxy(C₃-C₄)alkylgruppe oder einer C₂-C₄-Alkoxy-(C₁-C₂)-alkylgruppe sind oder R2 und R3 gemeinsam mit dem Stickstoffatom einen viergliedrigen bis achtgliedrigen heteroaliphatischen Ring bilden;
**R4** Wasserstoff oder eine C₁-C₆ Alkylgruppe darstellt;
**R5** gleich Wasserstoff, einer C₁-C₆-Alkylgruppe, einer ungesättigten C₂-C₆-Alkylgruppe, einer Hydroxy(C₂-C₄)alkylgruppe, einer Dihydroxy(C₃-C₄)alkylgruppe, einer Amino(C₂-C₄)alkylgruppe, einer Dimethylamino(C₂-C₄)alkylgruppe, einer Acetylamino(C₂-C₄)alkylgruppe, einer Methoxy(C₂-C₄)alkylgruppe, einer Ethoxy(C₂-C₄)alkylgruppe, einer C₁-C₄-Cyanoalkylgruppe, einer Carboxy(C₁-C₄)alkylgruppe oder einer Aminocarbonyl(C₁-C₄)alkylgruppe ist;
**R6,R7,R8,R9,R10** unabhängig voneinander Wasserstoff, ein Halogenatom (F,Cl, Br, J), eine Cyanogruppe, eine Hydroxygruppe, eine C₁-C₄-Alkoxygruppe, eine Hydroxy(C₂-C₄)alkoxygruppe, eine C₁-C₆-Alkylgruppe, eine C₁-C₄-Alkylthioethergruppe, eine Mercaptogruppe, eine Nitrogruppe, eine Aminogruppe, eine (C₁-C₄)Alkylaminogruppe, eine Hydroxy(C₂-C₄)alkylaminogruppe, eine Di(C₁-C₄)alkylaminogruppe, eine Di(hydroxy(C₂-C₄)alkyl)-aminogruppe, eine (Dihydroxy(C₃-C₄)alkyl)aminogruppe, eine (Hydroxy(C₂-C₄)alkyl)-(C₁-C₄)alkylaminogruppe, eine Trifluormethangruppe, eine -C(O)H-Gruppe, eine -C(O)CH₃-Gruppe, eine -C(O)CF₃-Gruppe, eine -Si(CH₃)₃-Gruppe, eine Hydroxy(C₂-C₄)alkylgruppe oder eine Dihydroxy(C₃-C₄)alkylgruppe bedeuten, oder zwei nebeneinanderliegende Reste R6 bis R10 eine -O-CH2-O-Brücke bilden.

2. Diaminopyrazol-Derivat nach Anspruch 1, **dadurch gekennzeichnet, dass** in der Formel (I) **R2** und **R3** gleich Wasserstoff sind und **R1** gleich einer C₁-C₄-Alkylgruppe, einer C₂-C₄-Hydroxyalkylgruppe, einer Benzylgruppe oder einer Methylbenzylgruppe ist.

3. Diaminopyrazol-Derivat nach Anspruch 1, **dadurch gekennzeichnet, dass** in der Formel (I) **R2, R3, R4** und **R5** gleich Wasserstoff sind und **R1** gleich einer C₁-C₄-Alkylgruppe, einer C₂-C₄-Hydroxyalkylgruppe, einer Benzylgruppe oder einer Methylbenzylgruppe ist.

4. Diaminopyrazol-Derivat nach Anspruch 1, **dadurch gekennzeichnet, dass** in der Formel (I) **R2, R3, R4** und **R5** gleich Wasserstoff sind und **R1** gleich einer C₁-C₄-Alkylgruppe, einer C₂-C₄-Hydroxyalkylgruppe, einer Benzylgruppe oder einer Methylbenzylgruppe ist, und mindestens einer der Reste R6 bis R10 gleich einer Hydroxygruppe oder einer Aminogruppe ist während die verbleibenden Reste R6 bis R10 gleich Wasserstoff sind.

5. Diaminopyrazol-Derivat nach einem der Ansprüche 1 bis 4, **dadurch gekennzeichnet, dass** es ausgewählt ist aus 4-{[4,5-Diamino-1-(2-hydroxyethyl)-1 H-pyrazol-3-ylmethyl]-amino}-phenol, 2-{4,5-Diamino-3-[(4-amino-phenylamino)-methyl]-pyrazol-1-yl}-ethanol, 2-(4,5-Diamino-3-phenylaminomethyl-pyrazol-1-yl)-ethanol, 2-Isopropyl-5-phenylaminomethyl-2H-pyrazol-3,4-diamin, 2-{4,5-Diamino-3-[(3-amino-phenylamino)-methyl]-pyrazol-1-yl}-ethanol, 2-{4,5-Diamino-3-[(4-amino-2-methylphenylamino)-methyl]-pyrazol-1-yl}-ethanol, 2-{4,5-Diamino-3-[(4-amino-3-methyl-phenylamino)-methyl]-pyrazol-1-yl}-ethanol, 2-(4,5-Diamino-3-{[4-amino-2-(2-hydroxyethyl)-phenylamino]-methyl)-pyrazol-1-yl)-ethanol, 2-(4,5-Diamino-3-{[4-amino-3-(2-hydroxyethyl)-phenylamino]-methyl}pyrazol-1-yl)-ethanol, 2-{4,5-Diamino-3-[(4-dimethylamino-phenylamino)-methyl]-pyrazol-1-yl}-ethanol, 2-[4,5-Diamino-3-(benzo[1,3]dioxol-5-ylaminomethyl)-pyrazol-1-yl]-ethanol, 4-Chloro-2-{[4,5-diamino-1-(2-hydroxyethyl)-1 H-pyrazol-3-ylmethyl]-amino}-phenol, 3-{[4,5-Diamino-1-(2-hydroxyethyl)-1H-pyrazol-3-ylmethyl]-amino}-phenol, 5-{[4,5-Diamino-1-(2-hydroxyethyl)-1H-pyrazol-3-ylmethyl]-amino}-2-methyl-phenol, 2-{4,5-Diamino-3-[(3,4-dimethoxy-phenylamino)-methyl]-pyrazol-1-yl}ethanol, 2-{[4,5-Diamino-1-(2-hydroxyethyl)-1H-pyrazol-3-ylmethyl]-amino}-4-nitro-phenol, 4-[(4,5-Diamino-1-isopropyl-1H-pyrazol-3-ylmethyl)-amino]-phenol, 3-[(4,5-Diamino-1-isopropyl-1H-pyrazol-3-ylmethyl)-amino]-phenol, 5-[(4,5-Diamino-1-isopropyl-1H-pyrazol-3-ylmethyl)-amino]-2-methyl-phenol, 5-[(3,4-Dimethoxy-phenylamino)-methyl]-2-isopropyl-2H-pyrazol-3,4-diamin, 2-[(4,5-Diamino-1-isopropyl-1H-pyrazol-3-ylmethyl)-amino]-4-nitro-phenol, 5-[(3-Amino-phenylamino)-methyl]-2-isopropyl-2H-pyrazol-3,4-diamin, 5-[(4-Amino-phenylamino)-methyl]-2-isopropyl-2H-pyrazol-3,4-diamin, 5-[(4-Amino-2-methyl-phenylamino)-methyl]-2-isopropyl-2H-pyrazol-3,4-diamin, 5-[(4-Amino-3-methylphenylamino)-methyl]-2-isopropyl-2H-pyrazol-3,4-diamin, 2-{5-Amino-2-[(4,5-diamino-1-isopropyl-1H-pyrazol-3-ylmethyl)-amino]-phenyl}-ethanol, 2-{6-Amino-3-[(4,5-diamino-1-isopropyl-1H-pyrazol-3-ylmethyl)-amino]-phenyl}-ethanol, 2-{4-Amino-2-[(4,5-diamino-1-isopropyl-1H-pyrazol-3-ylmethyl)-amino]-phenoxy}-ethanol und 5-[(4-Dimethylamino-phenylamino)-methyl]-2-isopropyl-2H-pyrazol-3,4-diamin, oder deren Salzen.

6. Mittel zum oxidativen Färben von Keratinfasern auf der Basis einer Entwicklersubstanz-Kupplersubstanz-Kombination, **dadurch gekennzeichnet, dass** es als Entwicklersubstanz mindestens ein Diaminopyrazol-Derivat der Formel (I) gemäß einem der Ansprüche 1 bis 5 enthält.

7. Mittel nach Anspruch 6, **dadurch gekennzeichnet, dass** das Diaminopyrazol-Derivat der Formel (I) in einer Menge von 0,005 bis 20 Gewichtsprozent enthalten ist.

8. Mittel nach Anspruch 6 oder 7, **dadurch gekennzeichnet, daß** die Kupplersubstanz ausgewählt ist aus N-(3-Dimethylamino-phenyl)harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxy-ethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1-(3-hydroxypropoxy)-benzol, 2,4-Diamino-1-(3-methoxy-propoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxy-ethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)-amino]-anilin, 3-[(2-Aminoethyl)-amino]-anilin, 1,3-Di(2,4-diamino-phenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxy-ethyl)amino]-2-methyl-phenol, 3-[(2-Hydroxy-ethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methylphenol, 3-[(2-Hydroxyethyl)-amino]-2-methyl-phenol, 2-Amino-3-hydroxypyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 1,3-Dihydroxy-2,4-dimethylbenzol, 3,4-Methylendioxy-phenol, 3,4-Methylendioxy-anilin, 5-[(2-Hydroxyethyl)-amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 4-Hydroxy-indol, 5-Hydroxy-indol, 6-Hydroxy-indol, 7-Hydroxy-indol und 2,3-Indolindion, oder deren Salzen.

9. Mittel einem der Ansprüche 6 bis 8, **dadurch gekennzeichnet, dass** es zusätzlich mindestens eine weitere Entwicklersubstanz und/oder mindestens einen direktziehenden Farbstoff enthält.

10. Mittel nach einem der Ansprüche 6 bis 9, **dadurch gekennzeichnet, dass** die Farbträgermasse mit dem Oxidationsmittel in einem Gewichtsverhältnis von 5:1 bis 1:3 vermischt wird.

11. Mittel nach Anspruch 10, **dadurch gekennzeichnet, dass** das gebrauchsfertige Oxidationsfärbemittel einen pH-Wert von 3 bis 11 aufweist.

12. Mittel nach einem der Ansprüche 6 bis 11, **dadurch gekennzeichnet, dass** es ein Haarfärbemittel ist.

## Claims

1. Diaminopyrazole derivative of the general formula (I) or its physiologically compatible, watersoluble salts with inorganic or organic acids 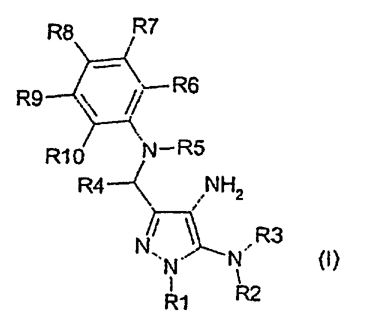 in which
**R1** is a C₁-C₆-alkyl group, a C₂-C₄-hydroxyalkyl group, a C₃-C₄-dihydroxyalkyl group or a C₂-C₆-alkoxy- (C₁-C₂) -alkyl group, a phenyl group, a benzyl group or a substituted benzyl group;
**R2 and R3,** independently of one another, are hydrogen, a C₁-C₆-alkyl group, a hydroxy(C₂-C₄)alkyl group, a dihydroxy(C₃-C₄)alkyl group or a C₂-C₄-alkoxy-(C₁-C₂)-alkyl group, or R2 and R3, together with the nitrogen atom, form a four-membered to eight-membered heteroaliphatic ring;
**R4** is hydrogen or a C₁-C₆-alkyl group;
**R5** is hydrogen, a C₁-C₆-alkyl group, an unsaturated C₂-C₆-alkyl group, a hydroxy(C₂-C₄)alkyl group, a dihydroxy (C₃-C₄) alkyl group, an amino (C₂-C₄) alkyl group, a dimethylamino(C₂-C₄)alkyl group, an acetylamino(C₂-C₄)alkyl group, a methoxy(C₂-C₄)alkyl group, an ethoxy(C₂-C₄)alkyl group, a C₁-C₄-cyanoalkyl group, a carboxy(C₁-C₄)alkyl group or an aminocarbonyl (C₁-C₄) alkyl group;
**R6, R7, R8, R9, R10,** independently of one another, are hydrogen, a halogen atom (F, Cl, Br, I), a cyano group, a hydroxyl group, a C₁-C₄-alkoxy group, a hydroxy(C₂-C₄)alkoxy group, a C₁-C₆-alkyl group, a C₁-C₄-alkylthioether group, a mercapto group, a nitro group, an amino group, a (C₁-C₄)alkylamino group, a hydroxy(C₂-C₄)alkylamino group, a di(C₁-C₄)alkylamino group, a di(hydroxy(C₂-C₄)alkyl)amino group, a (dihydroxy(C₃-C₄)alkyl)amino group, a (hydroxy(C₂-C₄)alkyl)(C₁-C₄)alkylamino group, a trifluoromethane group, a -C(O)H group, a -C(O)CH₃ group, a -C(O)CF₃ group, a -Si(CH₃)₃ group, a hydroxy(C₂-C₄)-alkyl group or a dihydroxy(C₃-C₄)alkyl group, or two adjacent radicals R6 to R10 form a -O-CH2-O-bridge.

2. Diaminopyrazole derivative according to Claim 1, **characterized in that**, in the formula (I), **R2** and **R3** are hydrogen and **R1** is a C₁-C₄-alkyl group, a C₂-C₄-hydroxyalkyl group, a benzyl group or a methylbenzyl group.

3. Diaminopyrazole derivative according to Claim 1, **characterized in that**, in the formula (I), **R2, R3, R4** and **R5** are hydrogen and **R1** is a C₁-C₄-alkyl group, a C₂-C₄-hydroxyalkyl group, a benzyl group or a methylbenzyl group.

4. Diaminopyrazole derivative according to Claim 1, **characterized in that**, in the formula (I), **R2, R3, R4** and **R5** are hydrogen and **R1** is a C₁-C₄-alkyl group, a C₂-C₄-hydroxyalkyl group, a benzyl group or a methylbenzyl group, and at least one of the radicals R6 to R10 is a hydroxyl group or an amino group, while the remaining radicals R6 to R10 are hydrogen.

5. Diaminopyrazole derivative according to one of Claims 1 to 4, **characterized in that** it is chosen from 4-{[4,5-diamino-1-(2-hydroxyethyl)-1H-pyrazol-3-ylmethyl]amino}phenol, 2-{4,5-diamino-3-[(4-aminophenylamino)methyl]pyrazol-1-yl}ethanol, 2-(4,5-diamino-3-phenylaminomethylpyrazol-1-yl)ethanol, 2-isopropyl-5-phenylaminomethyl-2H-pyrazole-3,4-diamine, 2-{4,5-diamino-3-[(3-aminophenylamino)methyl]pyrazol-1-yl}ethanol, 2-(4,5-diamino-3-[(4-amino-2-methylphenylamino)methyl]-pyrazol-1-yl}ethanol, 2-{4,5-diamino-3-[(4-amino-3-methylphenylamino)methyl]pyrazol-1-yl}ethanol, 2-(4,5-diamino-3-{[4-amino-2-(2-hydroxyethyl)-phenylamino]methyl}pyrazol-1-yl)ethanol, 2-(4,5-diamino-3-{[4-amino-3-(2-hydroxyethyl)phenylamino]methyl}pyrazol-1-yl)ethanol, 2-{4,5-diamino-3-[(4-dimethylaminophenylamino)methyl]pyrazol-1-yl}ethanol, 2-[4,5-diamino-3-(benzo[1,3]dioxol-5-ylaminomethyl}pyrazol-1-yl]ethanol, 4-chloro-2-{[4,5-diamino-1-(2-hydroxyethyl)-1H-pyrazol-3-ylmethyl]amino}phenol, 3-{[4,5-diamino-1-(2-hydroxyethyl)-1H-pyrazol-3-ylmethyl]amino}phenol, 5-{[4,5-diamino-1-(2-hydroxyethyl)-1H-pyrazol-3-ylmethyl]amino}-2-methylphenol, 2-{4,5-diamino-3-[(3,4-dimethoxyphenylamino)methyl]pyrazol-1-yl}-ethanol, 2-{[4,5-diamino-1-(2-hydroxyethyl)-1Hpyrazol-3-ylmethyl]amino}-4-nitrophenol, 4-[(4,5-diamino-1-isopropyl-1H-pyrazol-3-ylmethyl)amino]-phenol, 3-[(4,5-diamino-1-isopropyl-1H-pyrazol-3-ylmethyl)amino]phenol, 5-[(4,5-diamino-1-isopropyl-1H-pyrazol-3-ylmethyl)amino]-2-methylphenol, 5-[(3,4-dimethoxyphenylamino)methyl]-2-isopropyl-2H-pyrazole-3,4-diamine, 2-[(4,5-diamino-1-isopropyl-1H-pyrazol-3-ylmethyl)amino]-4-nitrophenol, 5-[(3-aminophenylamino)methyl]-2-isopropyl-2H-pyrazole-3,4-diamine, 5-[(4-aminophenylamino)methyl]-2-isopropyl-2H-pyrazole-3,4-diamine, 5-[(4-amino-2-methylphenylamino)methyl]-2-isopropyl-2H-pyrazole-3,4-diamine, 5-[(4-amino-3-methylphenylamino)methyl]-2-isopropyl-2H-pyrazole-3,4-diamine, 2-{5-amino-2-[(4,5-diamino-1-isopropyl-1H-pyrazol-3-ylmethyl)amino]phenyl}ethanol, 2-{6-amino-3-[(4,5-diamino-1-isopropyl-1H-pyrazol-3-ylmethyl)-amino]phenyl}ethanol, 2-{4-amino-2-[(4,5-diamino-1-isopropyl-1H-pyrazol-3-ylmethyl)amino]phenoxy}-ethanol and 5-[(4-dimethylaminophenylamino)-methyl]-2-isopropyl-2H-pyrazole-3,4-diamine, or salts thereof.

6. Agent for the oxidative dyeing of keratin fibres based on a developer substance-coupler substance combination, **characterized in that** it comprises at least one diaminopyrazole derivative of the formula (I) according to one of Claims 1 to 5 as developer substance.

7. Agent according to Claim 6, **characterized in that** the diaminopyrazole derivative of the formula (I) is present in an amount of from 0.005 to 20% by weight.

8. Agent according to Claim 6 or 7, **characterized in that** the coupler substance is chosen from N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1-(3-hydroxypropoxy)benzene, 2,4-diamino-1-(3-methoxypropoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]-aniline, 3-[(2-aminoethyl)amino]aniline, 1,3-di(2,4-diaminophenoxy)propane, di(2,4-diaminophenoxy) methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-2-methylphenol, 3-[(2-hydroxyethyl)amino]phenol, 3-[(2-methoxyethyl)-amino]phenol, 5-amino-2-ethylphenol, 2-(4-amino-2-hydroxyphenoxy)ethanol, 5-[(3-hydroxypropyl)-amino]-2-methylphenol, 3-[(2,3-dihydroxypropyl)-amino]-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 1,3-dihydroxy-2,4-dimethylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxy-indoline, 4-hydroxyindole, 5-hydroxyindole, 6-hydroxyindole, 7-hydroxyindole and 2,3-indoline-dione, or salts thereof.

9. Agent according to one of Claims 6 to 8, **characterized in that** it additionally comprises at least one further developer substance and/or at least one direct dye.

10. Agent according to one of Claims 6 to 9, **characterized in that** the colour carrier mass is mixed with the oxidizing agent in a weight ratio of from 5:1 to 1:3.

11. Agent according to Claim 10, **characterized in that** the ready-to-use oxidation dye has a pH of from 3 to 11.

12. Agent according to one of Claims 6 to 11, **characterized in that** it is a hair dye.

## Revendications

1. Dérivé de diaminopyrazole de formule générale (I) ou sels hydrosolubles physiologiquement acceptables d'un tel dérivé avec des acides organiques ou minéraux 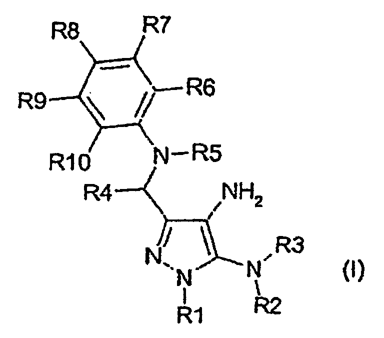 formule dans laquelle
**R1** représente un groupe alkyle en C₁-C₆, un groupe hydroxyalkyle en C₂-C₄, un groupe dihydroxyalkyle en C₃-C₄ ou un groupe alcoxy(C₂-C₆)-alkyle(C₁-C₂), un groupe phényle, un groupe benzyle ou un groupe benzyle substitué ;
**R2 et R3** représentent, indépendamment l'un de l'autre, un atome d'hydrogène, un groupe alkyle en C₁-C₆, un groupe hydroxyalkyle en C₂-C₄, un groupe dihydroxyalkyle en C₃-C₄ ou un groupe alcoxy(C₂-C₄)-alkyle(C₁-C₂), ou R2 et R3 forment ensemble avec l'atome d'azote un cycle hétéroaliphatique ayant de quatre chaînons ou à huit chaînons ;
**R4** représente un atome d'hydrogène ou un groupe alkyle en C₁-C₆ ;
**R5** représente un atome d'hydrogène, un groupe alkyle en C₁-C₆, un groupe alkyle en C₂-C₆ insaturé, un groupe hydroxyalkyle en C₂-C₄, un groupe dihydroxyalkyle en C₃-C₄, un groupe aminoalkyle(C₂-C₄), un groupe diméthylaminoalkyle(C₂-C₄), un groupe acétylaminoalkyle(C₂-C₄), un groupe méthoxyalkyle(C₂-C₄), un groupe éthoxyalkyle(C₂-C₄), un groupe cyanoalkyle(C₁-C₄), un groupe carboxyalkyle(C₁-C₄) ou un groupe aminocarbonyl-alkyle(C₁-C₄) ;
**R6, R7, R8, R9, R10** représentent, indépendamment les uns des autres, un atome d'hydrogène, un atome d'halogène (F, Cl, Br, I), un groupe cyano, un groupe hydroxy, un groupe alcoxy en C₁-C₄, un groupe hydroxy-alcoxy (C₂-C₄), un groupe alkyle en C₁-C₆, un groupe alkyl(C₁-C₄)thioéther, un groupe mercapto, un groupe nitro, un groupe amino, un groupe alkyl (C₁-C₄) amino, un groupe hydroxyalkyl(C₂-C₄)amino, un groupe dialkyl(C₁-C₄)amino, un groupe dihydroxyalkyl(C₂-C₄)amino, un groupe (dihydroxyalkyl(C₃-C₄))amino, un groupe (hydroxy-alkyl(C₂-C₄))-alkyl(C₁-C₄)amino, un groupe trifluorométhane, un groupe -C(O)H, un groupe -C(O)CH₃, un groupe -C(O)CF₃, un groupe -Si(CH₃)₃, un groupe hydroxyalkyle en C₂-C₄ ou un groupe dihydroxyalkyle en C₃-C₄, ou deux radicaux R6 à R10 adjacents forment un pont -O-CH₂-O-.

2. Dérivé de diaminopyrazole selon la revendication 1, **caractérisé en ce que** dans la formule (I) **R2** et **R3** représentent des atomes d'hydrogène et **R1** représente un groupe alkyle en C₁-C₄, un groupe hydroxyalkyle en C₂-C₄, un groupe benzyle ou un groupe méthylbenzyle.

3. Dérivé de diaminopyrazole selon la revendication 1, **caractérisé en ce que** dans la formule (I) R2, **R3, R4** et **R5** représentent des atomes d'hydrogène et **R1** représente un groupe alkyle en C₁-C₄, un groupe hydroxyalkyle en C₂-C₄, un groupe benzyle ou un groupe méthylbenzyle.

4. Dérivé de diaminopyrazole selon la revendication 1, **caractérisé en ce que** dans la formule (I) R2, **R3, R4** et **R5** représentent des atomes d'hydrogène et **R1** représente un groupe alkyle en C₁-C₄, un groupe hydroxyalkyle en C₂-C₄, un groupe benzyle ou un groupe méthylbenzyle, et au moins un des radicaux **R6** à **R10** représente un groupe hydroxy ou un groupe amino, tandis que les radicaux R6 à R10 restants sont des atomes d'hydrogène.

5. Dérivé de diaminopyrazole selon l'une quelconque des revendications 1 à 4, **caractérisé en ce qu'**il est choisi parmi le 4-{[4,5-diamino-1-(2-hydroxyéthyl)-1H-pyrazol-3-ylméthyl]-amino}-phénol, le 2-{4,5-diamino-3[(4-amino-phénolamino)-méthyl]-pyrazol-1-yl}-éthanol, le 2-(4,5-diamino-3-phénylaminométhyl-pyrazol-1-yl)-éthanol, la 2-isopropyl-5-phénylaminométhyl-2H-pyrazole-3,4-diamine, le 2-{4,5-diamino-3-[(3-amino-phénylamino)méthyl]-pyrazol-1-yl}-éthanol, le 2-{4,5-diamino-3-[(4-amino-2-méthylphénylamino)-méthyl]-pyrazol-1-yl}-éthanol, le 2-{4,5-diamino-3-[(4-amino-3-méthylphénylamino)-méthyl]-pyrazol-1-yl}-éthanol, le 2-(4,5-diamino-3-{[4-amino-2-(2-hydroxyéthyl)-phénylamino]-méthyl}-pyrazol-l-yl)-éthanol, le 2-(4,5-diamino-3-{[4-amino-3-(2-hydroxyéthyl)-phénylamino]-méthyl}pyrazol-1-yl)-éthanol, le 2-(4,5-diamino-3-[(4-diméthylamino-phénylamino)-méthyl]-pyrazol-1-yl}-éthanol, le 2-[4,5-diamino-3-(benzo[1,3]dioxol-5-ylaminométhyl)-pyrazol-1-yl]-éthanol, le 4-chloro-2-{[4,5-diamino-1-(2-hydroxyéthyl)-1H-pyrazol-3-ylméthyl]-amino}-phénol, le 3-{[4,5-diamino-1-(2-hydroxyéthyl)-1H-pyrazol-3-ylméthyl]-amino}-phénol, le 5-{[4,5-diamino-1-(2-hydroxyéthyl)-1H-pyrazol-3-ylméthyl]-amino}-2-méthyl-phénol, le 2-{4,5-diamino-3-[(3,4-diméthoxy-phénylamino)-méthyl]-pyrazol-1-yl}-éthanol, le 2-{[4,5-diamino-1-(2-hydroxyéthyl)-1H-pyrazol-3-ylméthyl]amino}-4-nitro-phénol, le 4-[(4,5-diamino-1-isopropyl-1H-pyrazol-3-ylméthyl)-amino]-phénol, le 3-[(4,5-diamino-1-isopropyl-1H-pyrazol-3-ylméthyl)amino]-phénol, le 5-[(4,5-diamino-1-isopropyl-1H-pyrazol-3-ylméthyl)amino]-2-méthyl-phénol, la 5-[(3,4-diméthoxy-phénylamino)-méthyl]-2-isopropyl-2H-pyrazole-3,4-diamine, le 2-[(4,5-diamino-1-isopropyl-1H-pyrazol-3-ylméthyl)-amino]-4-nitro-phényle, la 5-[(3-amino-phénylamino)-méthyl]-2-isopropyl-2H-pyrazole-3,4-diamine, la 5-[(4-amino-phénylamino)-méthyl]-2-isopropyl-2H-pyrazole-3,4-diamine, la 5-[(4-amino-2-méthyl-phénylamino)méthyl]-2-isopropyl-2H-pyrazole-3,4-diamine, la 5-[(4-amino-3-méthylphénylamino)-méthyl]-2-isopropyl-2H-pyrazole-3,4-diamine, le 2-{5-amino-2-[(4,5-diamino-1-isopropyl-1H-pyrazol-3-ylméthyl)-amino]-phényl}-éthanol, le 2-{6-amino-3-[(4,5-diamino-1-isopropyl-1H-pyrazol-3-ylméthyl)-amino]-phényl}-éthanol, le 2-{4-amino-2-[(4,5-diamino-1-isopropyl-1H-pyrazol-3-ylméthyl)-amino]-phénoxy}-éthanol et la 5-[(4-diméthylamino-phénylamino)-méthyl]-2-isopropyl-2H-pyrazole-3,4-diamine, ou leurs sels.

6. Composition pour la teinture par oxydation de fibres de kératine, à base d'une association développeur-coupleur, **caractérisée en ce qu'**elle contient en tant que développeur au moins un dérivé de diaminobenzène de formule (I) selon l'une quelconque des revendications 1 à 5.

7. Composition selon la revendication 6, **caractérisée en ce qu'**elle contient le dérivé de diaminopyrazole de formule (I) en une quantité de 0,005 à 20 % en poids.

8. Composition selon la revendication 6 ou 7, **caractérisée en ce que** le coupleur est choisi parmi la N-(3-diméthylaminophényl)urée, la 2,6-diaminopyridine, le 2-amino-4-[(2-hydroxyéthyl)-amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino}-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)benzène, le 2,4-diamino-1-(3-hydroxypropoxy)benzène, le 2,4-diamino-1-(3-méthoxypropoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]-aniline, le 4-amino-2-di[(2-hydroxyéthyl)amino]-1-éthoxybenzène, le 5-méthyl-2-(1-méthyléthyl)-phénol, la 3-[(2-hydroxyéthyl)amino]aniline, la 3-[(2-aminoéthyl)amino]aniline, le 1,3-di(2,4-diaminophénoxy)propane, le di(2,4-diaminophénoxy)-méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]-acétamide, le 5-[(2-hydroxyéthyl)amino]-2-méthylphénol, le 3-[(2-hydroxyéthyl)amino]phénol, 3-[(2-méthoxyéthyl)amino]phénol, le 5-amino-2-éthylphénol, le 2-(4-amino-2-hydroxyphénoxy)éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthyl-phénol, la 2-amino-3-hydroxypyridine, le. 5-amino-4-chloro-2-méthylphénol, le 1-naphtol, le 1,5-dihydroxynaphthalène, 1,7-dihydroxynaphthalène, le 2,3-dihydroxynaphthalène, le 2,7-dihydroxynaphthalène, l'acétate de 2-méthyl-1-naphtol, le 1,3-dihydroxybenzène, le 1-chloro-2,4-dihydroxybenzène, le 2-chloro-1,3-dihydroxybenzène, 1,2-dichloro-3,5-dihydroxy-4-méthylbenzène, le 1,5-dichloro-2,4-dihydroxybenzène, le 1,3-dihydroxy-2-méthylbenzène, le 1,3-dihydroxy-2,4-diméthylbenzène, le 3,4-méthylènedioxyphénol, la 3,4-méthylènedioxyaniline, le 5-[(2-hydroxyéthyl)-amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxy-indole, la 5,6-dihydroxy-indoline, le 4-hydroxy-indole, le 5-hydroxy-indole, le 6-hydroxy-indole, le 7-hydroxy-indole et la 2,3-indolinedione, ou leurs sels.

9. Composition selon l'une quelconque des revendications 6 à 8, **caractérisée en ce qu'**elle contient au moins un autre coupleur et/ou au moins un colorant direct.

10. Composition selon l'une quelconque des revendications 6 à 9, **caractérisée en ce que** la matière colorante est mélangée avec l'oxydant en un rapport en poids de 5:1 à 1:3.

11. Composition selon la revendication 10, **caractérisée en ce que** la composition de teinture par oxydation prête à l'emploi présente un pH de 3 à 11.

12. Composition selon l'une quelconque des revendications 6 à 11, **caractérisée en ce qu'**elle est une composition de teinture pour cheveux.
